# EUROPEAN PATENT SPECIFICATION

(11) **EP 0 845 449 B1**
(45) Date of publication and mention of the grant of the patent: **11.09.2002**
(21) Application number: 97203662.8
(22) Date of filing: 24.11.1997
(51) Int. Cl.: C07C 43/11, C07C 41/03, C11D 1/72

(54) **Ethoxylate mixture and a cleaning composition for hard surfaces containing the ethoxylate mixture**
Ethoxylatmischung und ein Reinigungsmittel für harte Oberflächen, eine enthaltendes Ethoxylatmischung
Mélange d'éthoxyle et composition de nettoyage pour surfaces dures contenant le mélange d'éthoxyle

(30) Priority: 27.11.1996 SE 9604349
(43) Date of publication of application: 03.06.1998
(73) Proprietor: AKZO NOBEL N.V., 6800 SB Arnhem (NL)
(72) Inventor: Sköld, Rolf, 44441 Stenungsund (SE)
(74) Representative: Andersson, Rolf

(56) References cited:
- EP-A- 0 046 582
- WO-A-94/11331
- Chemical Abstracts, volume 63, no 3, 2 August 1965, (Columbus, Ohio, US), S.M. Gurvich et al, "synthesis of some monoalkyl ethers of polyethylene and polypropylene glycols",, THE ABSTRACT No 2890, Zh. Organ. Khim. 1965, 1 3), 500-502
- STN International, File CA, Chemical Abstracts, volume 126, no.5, 3 February 1997 (Columbus Ohio, US), Moryama, Shigeki et al: "Detergents for cleaning offset printers", abstract no. 61919; & JP,A,08 283629 (TOYO INK MFG CO LTD) 961029
- STN International, file CA, Chemical Abstracts, volume 125, no. 10, 2 September 1996 (Columbus, Ohio, US), Kuki, Yukihiro et al: "Glycidyl- terminated ethylene glycol oligomer as reactive diluent for epoxy resins", abstract no. 115426; & JP,A,08 119955 (KYOEISHA KAGAKU KK) 960514
- STN International, File CA, Chemical Abstracts, volume 120, no. 20, 16 May 1994, Kondo, Masao: "Detergent compositions easily treatable after use" abstract no. 247860, & JP,A,05 287300 (ASAHI CHEM IND CO LTD) 931102
- STN International, File Ca, Chemical Abstracts, volume 109, no. 17, 24 October 1988 (Columbus, Ohio,US), Fujita, Kiyoshi et al: "Preparation of alkyleneglycol monoalkyl ethers", abstract no. 148882, & JP,A,62 289537 (KYOWA YUKA KK) 871216

## Description

The present invention relates to a mixture of ethoxylated 2-ethyl hexanol with a narrow distribution of ethyleneoxy units and a low content of unreacted 2-ethyl hexanol and use of the ethoxylate mixture in alkaline compositions for cleaning of hard surfaces.

In the cleaning of hard surfaces, it is a well-known problem that the cleaning tenside often has too high foaming, which results in a number of drawbacks. For instance, the cleaning effect is reduced by high foaming. Besides, high foaming may result in foaming over and increase the need of rinsing. Attempts have previously been made to solve these problems, for instance, by using as cleaning tensides low-foaming nonionic surfactants, such as ethoxylate of straight alcohols having 10-18 carbon atoms, the ethoxylates optionally being terminally blocked with lower alkyl groups or being further alkoxylated with one or more moles of propylene oxide and/or butylene oxide per mole of hydroxyl. The terminal blockings, however, result in a reduction of the cleaning effect and a lower degree of biological degradability. It is also known to add antifoaming compounds, such as monoalcohols. The above-described nonionic surfactants certainly have low foaming compared with other surfactants, such as anionic surfactants, but further improvements are desirable in order to satisfy the requirements that are placed on alkaline compositions for cleaning of hard surfaces.

It is also known to use lower branched alcohols. Thus, WO 94/11331 discloses that 2-propyl heptanol can advantageously be used to prepare ethoxylate having a good cleaning effect and low foaming.

The most easily accessible primary alcohol with branching in 2-position, 2-ethyl hexanol, has also been prepared for the preparation of ethoxylate for use in alkaline components intended for the cleaning of hard surfaces. In conventional ethoxylation by using alkali, such as KOH and/or NaOH, a reaction product having high contents of unreacted 2-ethyl hexanol is obtained. The presence of 2-ethyl hexanol in the ethoxylate promotes both an improved cleaning effect owing to its solubilising capacity and a reduced degree of foaming. However, the foaming of the ethoxylate, despite the presence of 2-ethyl hexanol, is too high, at the same time as the presence of 2-ethyl hexanol also in low contents results in a most unpleasant smell, which makes the 2-ethyl hexanol ethoxylate not being commercially used to any considerable extent.

Owing to 2-ethyl hexanol being easily accessible and easily biologically degradable, there is a desire to try to use an ethoxylate mixture based on 2-ethyl hexanol as surfactant in cleaning compositions for hard surfaces. This requires, however, that the foaming of the mixture can be reduced and the problem of smell can be eliminated while a good cleaning effect is achieved.

It has now surprisingly been found that it is possible to achieve this object by using as surfactant a mixture of ethoxylated 2-ethyl hexanol of the general formula 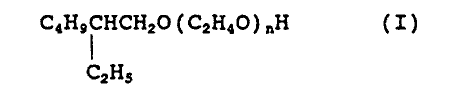 wherein n has an average value of 1.9-7, preferably 2.5-5, the ethoxylate containing 2-ethyl hexanol in a content of at most 0.5% by weight, preferably at most 0.2% by weight, the ethoxylate being obtained by the ethoxylation reaction being carried out in the presence of an ethoxylation catalyst, which yields a narrower distribution of the number of ethyleneoxy units than is obtained with KOH or NaOH as ethoxylation catalyst. The ethoxylation with a catalyst which gives a narrow ethyleneoxy distribution also results in a low content of 2-ethyl hexanol. This content is then further reduced by conventional treatment, for instance by distillation or evaporation, to a content below 0.5, which is so low as not to cause any disagreeable smell. From experience one would expect that the foaming of the ethoxylate mixture would increase significantly, since the content of 2-ethyl hexanol has been reduced to a considerable extent, but it has been found instead that the foaming capacity is reduced significantly while the cleaning capacity is maintained or improved.

This surprising effect will probably also be associated with the use of a catalyst, which yields a narrow distribution of the number of ethyleneoxy groups. Examples of suitable catalysts which yield a narrow distribution are zeolites, hydrotalcite, Ca(OH)₂, Ba(OH)₂, Sr(OH)₂, boron trifluoride, tin tetrachloride, aluminium tripropylene oxide and magnesium diethylene oxide. The reaction is carried out in a conventional manner in the presence of catalytic amounts, usually 0.5-3% by weight of the catalyst and in the absence of free water for reducing the amount of by-products at a temperature between 80° and 180°C.

The ethoxylate mixture according to the invention is suitable to be included in compositions for cleaning hard surfaces, such as for degreasing or washing. Particularly good results are achieved in connection with degreasing of painted or unpainted metal surfaces and glass surfaces. Good results have also been achieved when cleaning process equipment and premises in the foodstuff industry, for instance in the dairy industry, slaughter-house industry, fish industry and beer industry. Here the combination of low foaming and a good cleaning capacity in respect of protein and fatty dirt has been found to be of great value. Another field of application is the cleaning of sanitary equipment.

The present invention also relates to a cleaning agent, which in concentrated form contains
a) 1-10%, preferably 2-10% by weight of an ethoxylate mixture according to the invention,
b) 0.3-15%, preferably 1-10% by weight of a solubilising agent,
c) 0-30%, preferably 1-15% by weight of a complexing salt,
d) 0-20%, preferably 1-10% by weight of an alkalising agent or 0-50%, preferably 1-40% by weight of an acidifying agent, and
e) 40-98.7%, preferably 50-96% by weight of water.
As a rule, the concentrate has a pH of 1-13. When used for removal of fat, proteins or hydrophobic particulate soil, preferably alkaline concentrates having a pH of 8-13 are used, while for cleaning which comprises dissolution of lime or rust, concentrates having pH values of 2-6 are preferred. Before use, the concentrate is diluted with water 4-100 times its weight.

The solubilising agent is suitably present in the form of ionic or nonionic surfactants. Examples of suitable anionic solubilising agents are paratoluenesulphonic acid, phosphate esters, such as monoalkyl- and/or dialkylphosphate esters; cationic surfactants, such as quaternary mono- or difatty alkyl amine ethoxylates; nonionic surfactants, such as fatty alkyl ethanol amide ethoxylates and alkylglycosides; and amphoteric surfactants, such as alkali salts of alkyl amphopolycarboxy glycinate, alkyl aminoproprionate, alkyl amphopolycarboxy proprionate, alkyl amphocarboxy glycinate and alkyl iminodiproprionate or corresponding amphopolycarboxy compounds, in which at least one alkyl group has been replaced with an acyl group. Preferred solubilising compounds are those containing at least one alkyl group or acyl group having 8-18 carbon atoms.

Examples of suitable complexing salts are EDTA, NTA, citrate, normal potassium pyrophosphate, while the alkalising agents usually are metasilicate, disilicate, alkali, inorganic polyphosphates and inorganic carbonates. The alkalising agents are added in such an amount that the pH of the concentrate suitably is 8-13. As acidifying agent, use can be made of hydrochloric acid, citric acid, phosphoric acid and acetic acid. An acid cleaning agent usually contains no complexing salt or just a small amount thereof, for instance less than 5% by weight.

In addition to the above-mentioned components, the composition may also contain other cleaning surfactants than ethoxylate mixtures, such as anionic surfactants. Examples are alkyl sulphate, alkyl ether sulphate, alkyl benzene sulphonate, α-olefin sulphonate and alkyl glyceryl sulphonate, which contain a hydrocarbon residue having 8-20 carbon atoms. Moreover, the composition may also contain nonionic non-surfactant solubilising agents, enzymes, pH-controlling agents, bactericides, perfumes, colorants and viscosity-controlling additives and other components usually appearing in cleaning concentrates.

The invention will be further illustrated by way of the following Examples.

### Example 1

O.5 kmole of 2-ethyl hexanol and 1.5% by weight, based on the alcohol, of KOH or Ca(OH)₂ were charged into an ethoxylation reactor. The latter is a catalyst which gives a narrow distribution of ethylene oxide. The reactor was rinsed with nitrogen gas, whereupon the reactor was evacuated and the temperature increased to 100°C and ethylene oxide was charged continuously in a total amount of 1.00, 1.25, 1.50, 2.00 or 2.25 kmole. After completion of the reaction, the catalyst was neutralised. Part of the ethoxylate mixtures were then subjected to distillation for removal of unreacted 2-ethyl hexanol to contents below 0.2% by weight. The undistilled and distilled ethoxylates were then subjected to analysis by gas chromatography and mass chromatography. In respect of their composition, the following results were obtained.

**Table 1**

| Ethoxylate | mole EO/mole alcohol | Catalyst | Distillation | Content of 2-ethyl hexanol |
|---|---|---|---|---|
| 1 | 2.0 | KOH | Yes | 0.1 |
| 2 | 2.0 | KOH | No | 20 |
| 3 | 2.0 | Ca(OH)₂ | No | 10 |
| 4 | 2.0 | Ca(OH)₂ | Yes | 0.1 |
| 5 | 2.5 | KOH | Yes | 0.1 |
| 6 | 2.5 | KOH | No | 15 |
| 7 | 2.5 | Ca(OH)₂ | No | 8 |
| 8 | 2.5 | Ca(OH)₂ | Yes | 0.1 |
| 9 | 3.0 | KOH | Yes | 0.1 |
| 10 | 3.0 | KOH | No | 12 |
| 11 | 3.0 | Ca(OH)₂ | No | 5.0 |
| 12 | 3.0 | Ca(OH)₂ | Yes | 0.1 |
| 13 | 4.0 | KOH | Yes | 0.1 |
| 14 | 4.0 | KOH | No | 6 |
| 15 | 4.0 | Ca(OH)₂ | No | 2 |
| 16 | 4.0 | Ca(OH)₂ | Yes | 0.1 |
| 17 | 5.0 | KOH | Yes | 0.1 |
| 18 | 5.0 | KOH | No | 5 |
| 19 | 5.0 | Ca(OH)₂ | No | 1 |
| 20 | 5.0 | Ca(OH)₂ | Yes | 0.1 |

The ethoxylates according to the invention are numbered 4, 8, 12, 16 and 20.

### Example 2

A number of cleaning compositions intended for cleaning of hard surfaces were prepared. The basic formulation was as follows:
5% by weight of one of the alkoxylates numbered 1-20 in Table 1
6% by weight normal potassium pyrophosphate
4% by weight sodium metasilicate ^{·} 5 H₂O
2.5-6% by weight of quaternary fatty amine ethoxylate.
The amount was adjusted such that a clear concentrate was obtained.
Balance water.

The prepared concentrates, which had a pH of about 13, were then diluted with 100 parts by weight of water, and the foaming of the diluted solutions was then tested at 20°C and 40°C by vertically rotating a 500 ml measuring cylinder, which had been filled with 200 ml of one of the solutions, at 40 revolutions/min. The foam volume was then measured immediately and after 1 min. The following results were obtained.

**Table 2**

| Ethoxylate | Fatty amine ethoxylate % by weight | Foaming, ml | | | |
|---|---|---|---|---|---|
| | | 20°C | | 40°C | |
| | | 0 min | 1 min | 0 min | 1 min |
| 1 | 4 | 105 | 10 | 10 | 5 |
| 2 | 4.5 | 10 | 0 | 10 | 0 |
| 3 | 6 | 40 | 0 | 50 | 0 |
| 4 | 6 | 40 | 0 | 40 | 0 |
| 5 | 4 | 160 | 160 | 230 | 200 |
| 6 | 4.5 | 70 | 0 | 40 | 0 |
| 7 | 5.5 | 50 | 5 | 40 | 7 |
| 8 | 5 | 60 | 5 | 35 | 10 |
| 9 | 4 | 180 | 170 | 250 | 230 |
| 10 | 4.5 | 100 | 10 | 75 | 5 |
| 11 | 5 | 45 | 3 | 45 | 5 |
| 12 | 4 | 25 | 0 | 20 | 0 |
| 13 | 2.5 | 190 | 175 | 190 | 180 |
| 14 | 4 | 120 | 90 | 120 | 50 |
| 15 | 4 | 20 | 0 | 20 | 0 |
| 16 | 3.5 | 45 | 2 | 30 | 5 |
| 17 | 3 | 130 | 120 | 130 | 120 |
| 18 | 3 | 130 | 140 | 130 | 40 |
| 19 | 3 | 30 | 3 | 30 | 0 |
| 20 | 3 | 20 | 0 | 20 | 0 |

As appears from the results, formulations containing an ethoxylate mixture according to the invention, i.e. formulations 4, 8, 12, 16 and 20, yielded surprisingly low foam heights throughout, especially compared with formulations 1, 5, 9, 13 and 17, which contained ethoxylates having a low content of unreacted alcohol and prepared with KOH as catalyst.

### Example 3

In the same manner as in Example 2, various ethoxylate compositions and their effect on foaming were tested. The compositions, which had a pH of about 13, corresponded to the formulations in Example 2, with the exception that the quaternary fatty amine ethoxylate was replaced with 3-8% by weight of the amphoteric compound octyl iminodipropionate.

The following results were obtained.

**Table 3**

| Ethoxylate | Propionate, 40% % by weight | Foaming, ml | | | |
|---|---|---|---|---|---|
| | | 20°C | | 40°C | |
| | | 0 min | 1 min | 0 min | 1 min |
| 1 | 6 | 130 | 130 | 100 | 100 |
| 4 | 8 | 10 | 0 | 10 | 0 |
| 5 | 5 | 60 | 60 | 30 | 15 |
| 8 | 8 | 15 | 0 | 5 | 0 |
| 9 | 4 | 80 | 60 | 80 | 80 |
| 12 | 5.5 | 20 | 0 | 20 | 5 |
| 13 | 3.0 | 120 | 120 | 120 | 120 |
| 16 | 3.5 | 20 | 3 | 15 | 3 |
| 17 | 3 | 90 | 60 | 90 | 60 |
| 20 | 3 | 5 | 0 | 5 | 0 |

As appears from the results, the ethoxylate mixture according to the invention, i.e. 4, 8, 12, 16 and 20, has significantly better results than the corresponding ethoxylate mixtures obtained with KOH as catalyst.

### Example 4

In the same manner as in Example 3, foaming tests were performed except that the amphoteric compound in the formulation in Example 3 was replaced with the anionic compound decyl phosphate in an amount of 3.5-10% by weight. The concentrate had a pH of about 13.

The following results were obtained.

**Table 4**

| Ethoxylate | Phosphate, 43% % by weight | Foaming, ml | | | |
|---|---|---|---|---|---|
| | | 20°C | | 40°C | |
| | | 0 min | 1 min | 0 min | 1 min |
| 5 | 5 | 150 | 150 | 150 | 150 |
| 8 | 10 | 130 | 130 | 130 | 130 |
| 9 | 5 | 170 | 170 | 170 | 170 |
| 12 | 6 | 130 | 130 | 30 | 15 |
| 13 | 3.5 | 120 | 120 | 90 | 90 |
| 16 | 5.5 | 100 | 100 | 50 | 50 |
| 17 | 4.5 | 150 | 150 | 150 | 150 |
| 20 | 4.5 | 110 | 110 | 80 | 30 |

As appears from the results, the ethoxylate mixture according to the invention gave lower foaming than the reference products. The comparatively high foaming of the formulations depends on the fact that the solubilising agent, the decyl phosphate, is foaming.

### Examples 5-7

Vertically arranged, painted iron plates soiled with mineral oils, carbon black, salts and mud were subjected to the application of formulations according to Examples 2, 3 and 4, where the concentrate had been diluted with 19 parts of water. After 2 min, the plates were rinsed with water, whereupon the reflectance of the surface after soiling and cleaning was measured.

The following results were obtained. The reflectance after cleaning is indicated as % of the reflectance before soiling.

**Table 5**

| Ethoxylate | Reflectance, % | | |
|---|---|---|---|
| | Formulation Example 2 | Formulation Example 3 | Formulation Example 4 |
| 5 | 80.9 | 80.4 | 81.4 |
| 8 | 81.9 | 81.9 | 85.5 |
| 9 | 72.6 | 74.5 | 81.0 |
| 12 | 73.6 | 78.0 | 82.1 |
| 13 | 72.0 | 73.6 | 78.2 |
| 16 | 71.9 | 77.2 | 81.6 |
| 17 | 68.5 | 72.6 | 80.8 |
| 20 | 77.5 | 76.4 | 80.2 |

As appears from the results, formulations 8, 12, 16 and 20 according to the invention have an equivalent or better cleaning effect than the reference formulations.

## Claims

1. A mixture of ethoxylated 2-ethyl hexanol, **characterised in that** it has the general formula 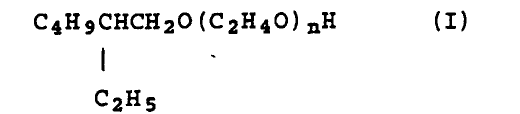 wherein n has an average value in the range of 1.9-7, the ethoxylate containing 2-ethyl hexanol in a content of at most 0.5% by weight and being obtained by an ethoxylation reaction carried out in the presence of an ethoxylation catalyst, which yields a narrower distribution of the number of ethyleneoxy units than is obtained with KOH or NaOH as ethoxylation catalyst.

2. A mixture as claimed in claim 1, **characterised in that** n has an average value in the range of 2.5-5.

3. A mixture as claimed in claim 1 or 2, **characterised in that** the content of 2-ethyl hexanol is at most 0.2% by weight.

4. A cleaning agent, **characterised in that**, in concentrated form, it contains
a) 1-10% by weight of an ethoxylate mixture as claimed in claim 1, 2 or 3,
b) 0.3-15% by weight of a solubilising agent,
c) 0-30% by weight of a complexing salt,
d) 0-20% by weight of an alkalising agent or 0-50% by weight of an acidifying agent, and
e) 40-98.7% by weight of water.

5. A cleaning agent as claimed in claim 4, **characterised in that** it contains
1-15% by weight of an alkalising agent as component d) and
1-10% by weight of component c)
and has a pH of 8-13.

6. A cleaning agent as claimed in claim 4 or 5, **characterised in that** the solubilising agent consists at least partly of a quaternary fatty alkyl amine ethoxylate.

7. A cleaning agent as claimed in claim 4, **characterised in that** it contains 1-40% by weight of an acidifying agent as component d) and 0-5% by weight of component c) and has a pH of 1-6.

8. Use of an ethoxylate mixture as claimed in claims 1-3 in compositions for cleaning of hard surfaces.

9. Use as claimed in claim 8 for degreasing of painted or unpainted metal surfaces.

10. Use as claimed claim 8 for cleaning of process equipment in the foodstuff industry.

## Patentansprüche

1. Mischung von ethoxyliertem 2-Ethylhexanol, **dadurch gekennzeichnet, dass** es die allgemeine Formel 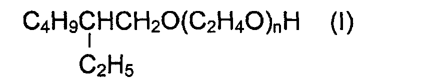 aufweist, worin n einen mittleren Wert im Bereich von 1,9 bis 7 aufweist, wobei das Ethoxylat 2-Ethylhexanol in einem Gehalt von höchstens 0,5 Gew.-% enthält und durch eine Ethoxylierungsreaktion erhalten wird, die in Anwesenheit eines Ethoxylierungskatalysators durchgeführt wird, der eine engere Verteilung der Anzahl an Ethylenoxyeinheiten ergibt als sie mit KOH oder NaOH als Ethoxylierungskatalysator erhalten wird.

2. Mischung wie in Anspruch 1 beansprucht, **dadurch gekennzeichnet, dass** n einen mittleren Wert im Bereich von 2,5 bis 5 aufweist.

3. Mischung wie in Anspruch 1 oder 2 beansprucht, **dadurch gekennzeichnet, dass** der Gehalt an 2-Ethylhexanol höchstens 0,2 Gew.-% beträgt.

4. Reinigungsmittel, **dadurch gekennzeichnet, dass** es in konzentrierter Form
a) 1 bis 10 Gew.-% einer Ethoxylat-Mischung wie in Anspruch 1, 2 oder 3 beansprucht,
b) 0,3 bis 15 Gew.-% eines Solubilisierungsmittels,
c) 0 bis 30 Gew.-% eines Komplexsalzes,
d) 0 bis 20 Gew.-% eines alkalisierenden Mittels oder 0 bis 50 Gew.-% eines Säuerungsmittels und
e) 40 bis 98,7 Gew.-% Wasser enthält.

5. Reinigungsmittel wie in Anspruch 4 beansprucht, **dadurch gekennzeichnet, dass** es 1 bis 15 Gew.-% eines alkalisierenden Mittels als Komponente d) und 1 bis 10 Gew.-% der Komponente c) enthält und einen pH-Wert von 8 bis 13 aufweist.

6. Reinigungsmittel wie in Anspruch 4 oder 5 beansprucht, **dadurch gekennzeichnet, dass** das Solubilisierungsmittel zumindest teilweise aus einem quaternären Fettalkylaminethoxylat besteht.

7. Reinigungsmittel wie in Anspruch 4 beansprucht, **dadurch gekennzeichnet, dass** es 1 bis 40 Gew.-% eines Säuerungsmittels als Komponente d) und 0 bis 5 Gew.-% der Komponente c) enthält und einen pH-Wert von 1 bis 6 aufweist.

8. Verwendung einer Ethoxylat-Mischung wie in den Ansprüchen 1 bis 3 beansprucht in Zusammensetzungen zur Reinigung von harten Oberflächen.

9. Verwendung wie in Anspruch 8 beansprucht zum Entfetten von lackierten oder unlackierten Metalloberflächen.

10. Verwendung wie in Anspruch 8 beansprucht zur Reinigung von Prozessanlagen in der Nahrungsmittelindustrie.

## Revendications

1. Mélange de 2-éthyl-hexanol éthoxylé, **caractérisé en ce qu'**il répond à la formule générale : 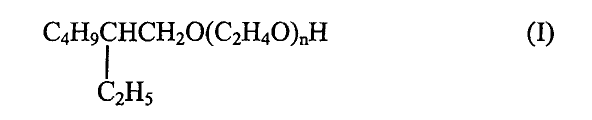 dans laquelle n a une valeur moyenne dans l'intervalle de 1,9 à 7, l'éthoxylate contenant du 2-éthyl-hexanol en une teneur d'au plus 0,5 % en poids et étant obtenu par une réaction d'éthoxylation conduite en présence d'un catalyseur d'éthoxylation, qui résulte en une distribution plus étroite du nombre de motifs éthylènoxy que celle obtenue avec KOH ou NaOH comme catalyseur d'éthoxylation.

2. Mélange selon la revendication 1, **caractérisé en ce que** n a une valeur moyenne dans l'intervalle de 2,5 à 5.

3. Mélange selon la revendication 1 ou 2, **caractérisé en ce que** la teneur en 2-éthyl-hexanol est d'au plus 0,2 % en poids.

4. Agent de nettoyage, **caractérisé en ce que**, sous forme concentrée, il contient :
a) 1-10 % en poids d'un mélange d'éthoxylates selon la revendication 1, 2 ou 3;
b) 0,3-15 % en poids d'un agent de solubilisation;
c) 0-30 % en poids d'un sel de complexation;
d) 0-20 % en poids d'un agent alcalinisant ou 0-50 % en poids d'un agent acidifiant; et
e) 40-98,7 % en poids d'eau.

5. Agent de nettoyage selon la revendication 4, **caractérisé en ce qu'**il contient :
1-15 % en poids d'un agent alcalinisant comme composant d), et
1-10 % en poids du composant c)
et a un pH de 8-13.

6. Agent de nettoyage selon la revendication 4 ou 5, **caractérisé en ce que** l'agent de solubilisation est constitué, au moins en partie, d'un éthoxylate d'amine d'alkyle gras quaternaire.

7. Agent de nettoyage selon la revendication 4, **caractérisé en ce qu'**il contient 1-40 % en poids d'un agent acidifiant comme composant d) et 0-5 % en poids du composant c), et a un pH de 1-6.

8. Utilisation d'un mélange d'éthoxylates selon les revendications 1-3 dans des compositions pour le nettoyage des surfaces dures.

9. Utilisation selon la revendication 8 pour le dégraissage des surfaces métalliques peintes ou non peintes.

10. Utilisation selon la revendication 8 pour le nettoyage d'équipement de traitement dans l'industrie alimentaire.
